Europäisches Patentamt

European Patent Office

Office européen des brevets

(11) Publication number: **0 190 719**
**A2**

# EUROPEAN PATENT APPLICATION

(21) Application number: **86101419.9**

(22) Date of filing: **04.02.86**

(51) Int. Cl.⁴: **A 61 B 17/34**

(30) Priority: **08.02.85 JP 23145/85**
**30.10.85 JP 165815/85 U**

(43) Date of publication of application:
**13.08.86 Bulletin 86/33**

(84) Designated Contracting States:
**DE FR GB**

(71) Applicant: **Fukutome, Takero**
**4-10 Wakagidai 1-chome Fukuma-cho**
**Munakata-gun Fukuoka-ken(JP)**

(71) Applicant: **Hayashi Medical Electronics Co. Ltd.**
**7-11 Arima 2 chome Miyamae-ku**
**Kawasaki-shi Kanagawa-ken(JP)**

(72) Inventor: **Fukutome, Takero**
**4-10 Wakagidai 1-chome, Fukuma-cho**
**Munakata-gun, Fukuoka-ken(JP)**

(72) Inventor: **Matsumoto, Hiroji**
**2703-14 Arima, Miyamae-ku**
**Kawasaki-shi, Kanagawa-ken(JP)**

(72) Inventor: **Koyama, Yoshimasa**
**8-13 Minami-ikuta 5-chome, Tama-ku**
**Kawasaki-shi, Kanagawa-ken(JP)**

(72) Inventor: **Sugawara, Masuo**
**8-20 Nishi-Sumiyoshi**
**Tokorozawa-shi, Saitama-ken(JP)**

(74) Representative: **Patentanwälte TER MEER - MÜLLER -**
**STEINMEISTER**
**Mauerkircherstrasse 45**
**D-8000 München 80(DE)**

(54) **Puncturing apparatus.**

(57) A transmission transducer (9; 34) produces an ultrasonic
wave and applies it to the blood vessel (12), and a reception
transducer (9; 52) provided at a puncturing needle (1)
receives the ultrasonic wave reflected by the blood vessel
(12) through the puncturing needle (1). When the reception
transducer (9; 52) receives the reflected ultrasonic wave, the
puncturing needle (1) faces the blood vessel (12)

FIG. 4

## PUNCTURING APPARATUS

### BACKGROUND OF THE INVENTION

The present invention relates to a puncturing apparatus for inserting a puncturing needle into a blood vessel correctly, and especially to a puncturing apparatus which is preferable to be applied to a case that a position of a blood vessel under skin, such as an artery or a vein under a collarbone, cannot be detected by palpation.

Recently, it is used for various kinds of examinations or therapies to insert a puncturing needle provided with a cannula, a cathether or the like into a blood vessel and leave the cannula or the cathether in the blood vessel by extracting the needle. It is important to insert the puncturing needle in the desired blood vessel correctly in the above-described case or a normal injection. It is somehow done to pierce correctly the blood vessel positioned at the exterior of the body, however, it is true to slightly reduce the probability that an expert pierces correctly the invisible blood vessel inside the body even if he knows the position thereof.

An apparatus is proposed to search the position of the blood bessel inside the body from the exterior thereof by detecting a reflected pulse waveform of a ultrasonic wave from the blood stream as a sound or a visible waveform by a use of Doppler effect of the ultrasonic wave. However, it is difficult for this apparatus to detect correctly the position of the blood vessel provided deeply in the body. In addition, there is no directly coupling relation between this apparatus and the puncturing needle.

According to the prior art, it is very difficult to insert a puncturing needle correctly to a blood vessel within the body, and various kinds of dangers may occur when puncturing an improper location. Thus, it is eagered to establish a method of inserting the puncturing needle to a desired blood vessel correctly and certainly.

It is an object of the present invention to provide a puncturing apparatus which overcome the aforementioned disadvantages of the prior art.

It is another object of the present invention to provide a puncturing apparatus having a detector at a tip of a puncturing needle to detect a blood vessel.

It is a further object of the present invention to provide a puncturing apparatus which is easy to detect the blood vessel and is able to detect the desired blood vessel rapidly even for an operator who has no experience in clinic.

Other objects and advantages of the present invention will become apparent to those having ordinary skill in the art when taken in conjunction with the accompanying drawings.

SUMMARY OF THE INVENTION

In the present invention, the blood stream detecting apparatus using Doppler effect (which will be referred to only probe hereinafter) has been placed on the market and put to practical use. This is an apparatus which detects a position of a blood stream, namely, a position of a blood vessel by directing ultrasonic wave to the blood vessel, and catching a frequency variation of the ultrasonic wave reflected by blood-corpuscles flowing through the blood vessel as a waveform or a sound waveform with an oscillograph or the like, the frequency variation resulting from Doppler effect based on a speed variation of the blood stream through the blood vessel. Since the waveform based on an artery stream is very different from the waveform based on a vein stream, these streams can be distinguished from each other. In this invention, the puncturing needle is defined as all puncturing needles used generally for various objects.

The liquor receiving side of the puncturing needle is coupled to the cylinder to observe a counter current of the blood for confirming that the needle is pierced to the blood vessel.

The present invention is characterized in that the sensor tip portion of the probe is positioned at the liquor receiving side of the puncturing needle, and a path of the transmitted and received ultrasonic wave of the probe is formed between the sensor tip and the tip of the puncturing needle. In other words, a ultrasonic shelter should not be provided between a ultrasonic transmission portion of the sensor tip and the tip of the puncturing needle. The shelter is, for example, gas, such as air or a hard solid, such as a metal. The liquor, gel, soft part tissue of the body or the like is a material which penetrates the ultrasonic wave very well. In general, the liquor is filled up to couple between the tip of the puncturing needle and the sensor tip. This liquor should not be injurious to the body, because it touches the blood of the blood vessel. This liquor is, for example, physiological saltwater, general injection liquor or the like.

The sensor tip of the probe is provided so as to face the tip of the puncturing needle; otherwise it is necessary to change the path of the ultrasonic wave by changing the direction of the ultrasonic wave to direct the ultrasonic wave to the needle tip. The ultrasonic wave has a straight characteristic with respect to normal sound.

The sensor tip may touch the liquor directly, however, in a practical puncturing device, it is preferable to provide a partition such that the sensor tip does not touch the liquor directly because of sterilization and desinfection. Of course, this partition should penetrate the ultrasonic wave, and almost all of so-called high molecular materials can be used for this purpose.

In this invention, what the liquor is infused from the sensor tip through the tip of the puncturing needle includes a case that the partition penetrating the ultrasonic wave is provided between the sensor tip and the liquor. In this instance, it is necessary to avoid to lie an ultrasonic shield layer, such as air layer when touching the sensor tip to the partition surface.

Not only do the puncturing needles include an injection sting for a general liquid medicine, but they include a puncturing needle for inserting a catheter into the blood vessel to examine or treat a heart or other intestines.

In addition, the present invention constructs a puncturing apparatus by separating it to a transmission probe and a reception probe. According to the present invention, a puncturing apparatus comprises a puncturing device, and a non-directional transmission probe having a transmission tranducer, wherein said puncturing device includes an adapter provided at an end of a puncturing needle, an injector and a reception probe are removably mounted to said adapter, and said reception probe includes a reception transducer to receive ultrasonic wave transmitted from said transmission probe.

According to this construction, when the ultrasonic wave output from the transmission probe enters the body, the output ultrasonic wave is radiated. When the ultrasonic wave collides with the blood vessel, it is reflected to the skin surface. Since the reflected wave appears within a predetermined area of the skin surface, the desired blood vessel can be detected only by inserting the puncturing device to any skin surface of the predetermined area and determining the direction thereof to detect the audio sound.

BRIEF DESCRIPTION OF THE DRAWINGS

FIG. 1 is a cross-sectional view of a first embodiment according to the present invention;

FIGs. 2, 3A and 3B shows cross-sectional views of a blood vessel for explaining the operation of FIG. 1;

FIG. 4 is a side view of a second embodiment according to the present invention;

FIG. 5 is a cross-sectional side view of a transmission probe used in FIG. 4;

FIG. 6 is a cross-sectional side view of a reception probe used in FIG. 4; and

FIG. 7 is a block diagram of a circuit used in FIG. 4.

DETAILED DESCRIPTION OF THE PREFERRED EMBODIMENTS

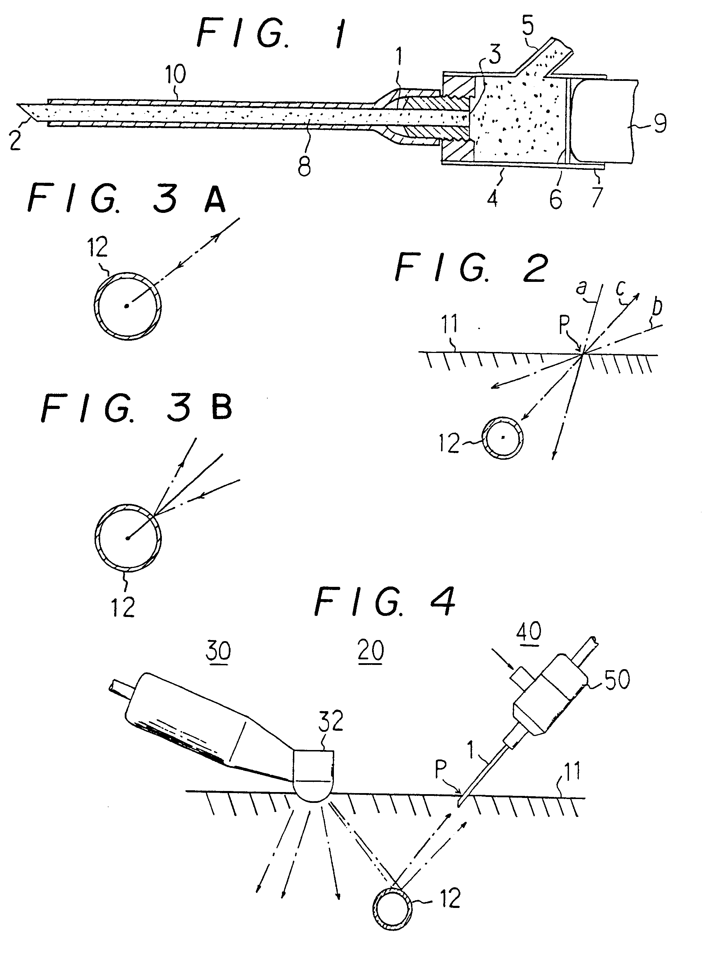

Reffering to FIG. 1, there is shown a cross-sectional side view of a puncturing apparatus according to a first embodiment of the present invention. In the drawing, a reference number 1 indicates a puncturing needle, 2:puncturing tip, 3:end portion to receive liquor, 4:cylinder portion coupled to the end portion, 5:branch tube provided at the cylinder portion to receive the liquor, 6:partition provided at the upper portion of the cylinder, 7:upper portion of the cylinder to couple to the sensor tip of the probe and 8:ultrasonic coupling medium (injection liquor).

The puncturing apparatus of the present invention is completed by the above-described elements, but the drawing further includes the sensor tip 9 of the probe (ultrasonic probe) and a cannula 10. A guide wire or a catheter is used frequently instead of the cannula within the needle.

This puncturing apparatus detects the position of the blood vessel by a use of ultrasonic wave, wherein a cylinder portion 4 is removably mounted to an end side 3 of a puncturing needle 1 opposite to an edge 2, a branch tube 5 is provided at the cylinder portion 4 to mount an injector (not shown), and a ultrasonic probe 9 is provided at an upper portion of the cylinder portion 4 on the center axis of the puncturing needle 1. The cylinder portion 4 is filled up with physiological saltwater 8 acting as ultrasonic coupling medium. A reference number 10 indicates a cannula (or a catheter).

In an operation of the puncturing apparatus constructed as described hereinbefore, since a ultrasonic transducer for both of transmission and reception is provided in parallel with a partition 6 in the ultrasonic probe 9, the transmitted ultrasonic wave goes straight through the cylinder portion 4 and the puncturing needle 1. If the reflected ultrasonic wave has a reflection angle same as the transmitted ultrasonic wave (i.e., if an input angle is equal to an output angle), the reflected ultrasonic wave will input to the ultrasonic probe 9 through the same transmission path of the output ultrasonic wave. When the ultrasonic wave is applied to the blood vessel, a frequency of the ultrasonic wave is changed by a blood stream speed of the blood flowing through the blood vessel. In other words, the received ultrasonic wave is effected by Doppler effect.

As described hereinbefore, the frequency variation based on the speed variation of the blood stream flowing through the blood vessel is converted into an audio signal. The piercing direction of the puncturing needle is determined in auditory sense in accordance with an existence of the audio signal or a modulation thereof. Thus, the desired blood vessel is pierced by the puncturing needle quickly and correctly. Since the above-described ultrasonic probe 9 uses the transducer for both the transmission and the reception, the desired blood vessel will be found by using this apparatus as follows:

In FIG. 2, a reference number 11 indicates a skin surface and a P indicates a piercing point to a blood vessel 12 positioned deeply in the skin. If the piercing direction of the puncturing needle 1 is an arrow a or b, the reflected ultrasonic wave from the blood vessel 12 cannot be detected. The ultrasonic Doppler probe 9 can detect the reflected wave only for a piecing direction c. In other words, only when the piercing direction coincides with the center point of the blood vessel 12 as shown in FIG. 3A, the direction of the output ultrasonic wave coincides with the direction of the input ultrasonic wave to the puncturing needle 1, and the reflected wave from the blood vessel 12 reaches the ultrasonic probe 9 through the puncturing needle 1. At this time, a signal effected by the Doppler effect is output as an audio sound, i.e., the reflected wave can be detected.

The above-described puncturing apparatus cannot detect the position of the blood vessel, when the direction of the input ultrasonic wave as the reflected wave from the blood vessel does not coincide with that of the output ultrasonic wave. For example, in a case that the piercing direction is shown in FIG. 3B, the reflected wave will not input to the puncturing needle 1, because the reflection direction of the ultrasonic wave differs from the incidence angle. Thus, the reflected wave cannot be detected, even if the ultrasonic wave is applied to the blood vessel 12. In this instance, the existence of the blood vessel cannot be confirmed.

Therefore, it is difficult for the puncturing apparatus of FIG. 1 to catch the reflected wave by piercing the skin surface 11 with the puncturing needle 1 and applying the ultrasonic wave, and it is necessary to change the piercing direction of the puncturing needle 1 many times. This puncturing apparatus can detect the blood vessel 12 in a short time in comparison with a prior art, but it is not enough to detect the desired blood vessel correctly and rapidly. This results from that the ultrasonic Doppler probe 9 uses the single transducer for both the transmission and the receiption.

A second embodiment of the present invention provides a puncturing apparatus which is easy to detect the blood vessel and is able to detect the desired blood vessel rapidly even for an operator who has no experience in clinic. This second embodiment of the puncturing apparatus according to the present invention will be discussed in detail by reference to FIG. 4.

FIG. 4 shows the concept of the puncturing apparatus 20 according to the present invention, wherein the puncturing apparatus 20 consists of a puncturing device 40 and a transmission probe 30. As is described hereinafter, a reception probe 50 is attached removably to one end of the puncturing needle 1 in the puncturing device 40. The transmission probe 30 pushes the skin surface 11 where the blood vessel 12 to be pierced by the puncturing needle 1 may be located. Since the transmission probe 30 is non-directional, the ultrasonic wave radiates when the transmission probe 30 applies the ultrasonic wave to the inside of the skin.

If there is the blood vessel 12 adjacent to the transmission probe 30, the ultrasonic wave applied to the blood vessel 12 will be reflected certainly. Thus, the reflected wave of the ultrasonic wave applied to the upper surface of the blood vessel 12 will reach the skin surface 11. It is possible to input the reflected wave of the ultrasonic wave to the reception probe attached to the puncturing device 40 only by piercing the destination area of the skin surface 11 with the puncturing needle 1 provided at the puncturing device 40 and changing the piercing direction thereof slightly, because the destination area of the reflected wave on the skin surface 11 is relatively large.

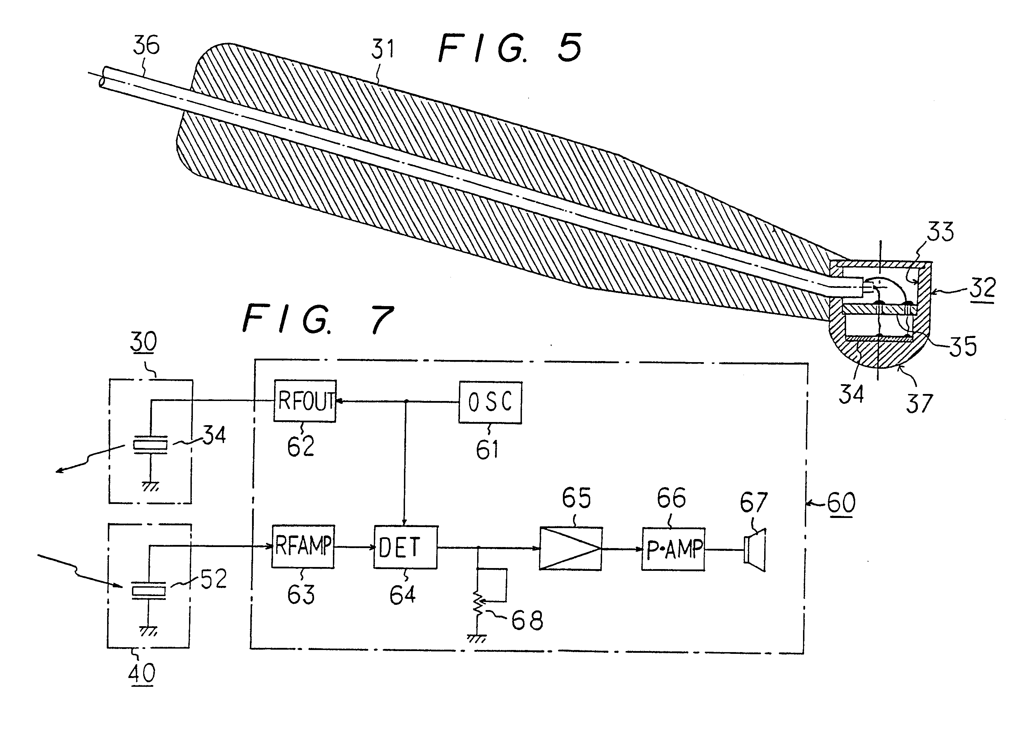

In the transmission probe 30, as shown in FIG. 5, a housing 32 is provided at one end of a bar-shaped handle portion 31, an inside 33 of the housing 32 is a cave, and a transmission transducer 34 is fixed to the bottom portion of the cave 33. In this embodiment, the transmission transducer 34 is a disk-shape, and an oscillation frequency thereof is a continuous frequency of 5 MHz. A reference number 35 indicates a terminal mounting board for the transmission transducer 34, and a 36 indicates a lead wire thereof.

The outside of the housing 32 including the transmission transducer 34, namely, a touching surface 37 for the skin is spherical as shown in the drawing. The ultrasonic wave radiated from the transmission transducer 34 is output in the normal direction with respect to the touching surface 37 of the spherical shape, so that the non-directional transmission probe 30 is established. The size of the touching surface 37 should be determined after consideration of the therapy of the patient. In this embodiment, the diameter and curvature of the touching surface are selected to approximate 15 mm and 8 mm, respectively.

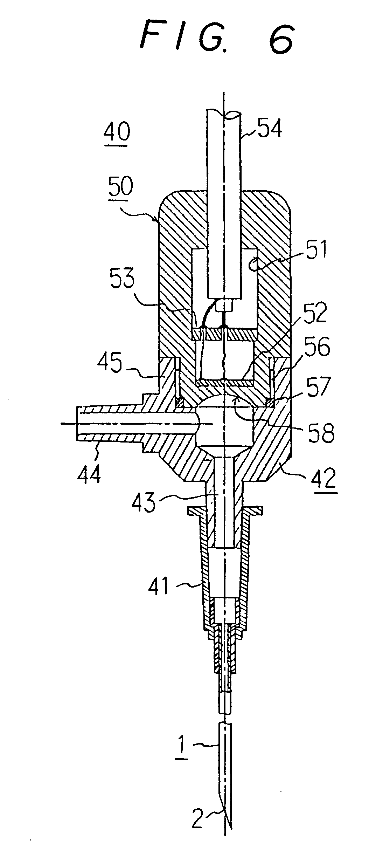

FIG. 6 shows one example of the puncturing device 40. One end of the puncturing needle 1 opposite to the edge 2 is mounted removably to an adapter 42 via an auxiliary adapter 41, and the inside of the adapter 42 is a cave to form a liquor path 43. A branch path 44 is coupled to the liquor path 43, and an injector (not shown) is mounted to the branch path 44. A cylinder of the injector may be directly mounted to the branch path 44, or a bender tube may be inserted therebetween.

The branch path 44 may be an L-shape to be bent upward, and the shape thereof may be modified if necessary. A coupling portion 45 is formed at the upper portion of the adapter 42 which is positioned on an extended line of the center axis of the puncturing needle 1, and the reception probe 50 is coupled removably to the coupling portion 45. The reception probe 50 is fixed to the adapter 42 with screws. A reference number 56 indicates the screws provided to them.

The inside of the reception probe 50 is a cave, and a reception transducer 52 is pased to the bottom portion 50a of the cave 51. The reception transducer 52 has a disk-shape. The outer end surface of the bottom portion 50a of the reception probe 50 has a spherical surface 58 to converge the received ultrasonic wave easily. Thus, an S/N ratio is improved. Reference numbers 53 and 54 represent a terminal plate and a lead wire, respectively.

The injector is mounted to the branch path 44 so as to seal them, and the reception probe 50 is mounted to the coupling portion 45 so as to seal them. In the drawing, a packing 57 is provided on the bottom portion of the coupling portion 45. The liquor path 43 is filled up with physiological saltwater. The puncturing needle 1, the auxiliary adapter 41, the adapter 42 and the injector are disposable and will not be used again, because it is troublesome to sterilize them after using them. Of course, it is possible to use them again if they are sterilized.

For detecting the desired blood vessel 12 with the puncturing apparatus constructed as described hereinbefore, the ultrasonic wave is applied to the blood vessel 12 under a condition that the transmission probe 30 is pushed to the skin surface 11 as shown in FIG. 4, a proper position of the skin surface 11 is pierced with the puncturing needle 1, and the existence of the audio sound is checked. If the puncturing needle 1 is positioned in the direction of the reflected ultrasonic wave, the reception transducer 52 of the reception probe 50 receives the reflected wave through the puncturing needle 1.

When the reception transducer 52 receives the reflected wave, the blood stream condition can be determined in accordance with the audio sound.  The puncturing needle 1 reaches the blood vessel 12 by further inserting the puncturing needle 1 at a position where the audio sound is the maximum.  After the blood vessel 12 is pierced correctly, the blood flows backward in the puncturing needle 1.  Thus, the piercing condition can be confirmed, and the liquid medicine is infused.

FIG. 7 shows one example of a circuit 60 which converts the reflected wave effected by the Doppler effect to the audio sound.  An oscillator 61 oscillates the sequence wave of 5 MHz as described hereinbefore, and the output signal therefrom is applied to the transmission transducer 34 through a radio frequency (RF) output circuit 62.  The reflected ultrasonic wave is received by the reception tranducer 52, and output signal therefrom is applied through a RF amplifier 63 to a detector circuit 64 which detects the reflected wave effected by the Doppler effect and converts it to the audio frequency.  The detected output signal is applied through an amplifier 65 and a power amplifier 66 to a speaker 67 so that the reflected wave is converted to the audio sound.  A variable resistor 68 is for adjusting the sound value.  In the embodiment of FIG. 7, if the detected output is applied to a waveform observation apparatus, such as an oscilloscope, the blood stream waveform can be observed simultaneously.

According to the present invention, the puncturing needle can be pierced correctly to a desired blood vessel positioned deeply in the body at a time, so that various risks of an improper piercing can be avoided. Moreover, the puncturing device of the present invention can be manufactured by modifying a conventional puncturing device slightly, and it is very practical. In addition, the present invention device can detect the blood vessel easily because the puncturing apparatus consists of the puncturing device 40 and the non-directional transmission probe 30. Therefore, it is possible for even an operator having less experience in clinic to detect the desired blood vessel rapidly and apply proper treatment and therapy.

While we have shown and described herein the preferred embodiments of our invention, it will be apparent to those skilled in the art that many changes and modifications may be made without departing from our invention in its broader aspects. For example, the shape of the probe may be modified to be thin so as to pass through the needle. However, the object of the present invention can be established by the probe positioned as shown in FIG. 1 if the liquor couples them. Therefore, the scope of the present invention should be determined only by the following claims.

CLAIMS:

1. A puncturing apparatus, characterized by

a transmission transducer (9;34) for producing an ultrasonic wave;

a puncturing needle (1);

an adapter (4;42) having one end coupled to said puncturing needle (1); and

a reception transducer (9;52) provided at the other end of said adapter (4;42), said reception transducer (9;52) receiving the ultrasonic wave.

2. A puncturing apparatus according to claim 1, characterized in that said adapter (4;42) includes a cave to receive liquid medicine.

3. A puncturing apparatus according to claim 1 or 2, characterized in that said reception transducer (9;52) is removably mounted to said adapter (4;42).

4. A puncturing apparatus according to one of the claims 1 to 3, characterized in that a coupling portion (7;45) is formed at the other end of said adapter (4;42).

5. A puncturing apparatus according to claim 4, characterized in that a bottom portion (6;50a) of said coupling portion (7;45) is a partition to touch said reception transducer (9;52).

6. A puncturing apparatus according to claim 5, characterized in that said bottom portion (6;50a) has a spherical shape to  converge the ultrasonic wave.

7. A puncturing apparatus according to claim 1, characterized in that said transmission transducer (9;34) and said reception transducer (9;52) are constructed by a single transducer.

8. A puncturing apparatus according to claim 1, characterized in that said transmission transducer (34) is mounted in a transmission probe (30) and said reception (52) is mounted in a reception probe (50).

9. A puncturing apparatus according to claim 8, characterized in that an exterior surface (37) of said transmission probe (30) has a spherical shape.

0190719

# FIG. 1

# FIG. 3 A

# FIG. 2

# FIG. 3 B

# FIG. 4

# FIG. 5

# FIG. 7

Takero Fukutome
Hayashi Medical
P-7E
2/3

0190719

# F I G. 6